# EUROPEAN PATENT SPECIFICATION

(11) **EP 1 200 099 B1**
(45) Date of publication and mention of the grant of the patent: **04.06.2003**
(21) Application number: 00949297.6
(22) Date of filing: 10.07.2000
(51) Int. Cl.: A61K 31/70, A61P 35/00, A61K 31/505

(54) **SYNERGISTIC COMPOSITION COMPRISING DAUNORUBICIN DERIVATIVES AND ANTIMETABOLITE COMPOUNDS**
SYNERGISTISCHE ZUSAMMENSETZUNG, DIE DAUNORUBICIN DERIVATE UND ANTIMETABOLITE ENTHÄLT
COMPOSITION A SYNERGIE COMPORTANT DES DERIVES DAUNORUBICINES ET DES COMPOSES ANTIMETABOLITES

(30) Priority: 19.07.1999 GB 9916882
(43) Date of publication of application: 02.05.2002
(73) Proprietor: Pharmacia Italia S.p.A., 20152 Milano (IT)
(72) Inventor: GERONI, Maria, Cristina, I-20149 Milan (IT); RIPAMONTI, Marina, I-20162 Milan (IT); CARUSO, Michele, I-20131 Milan (IT); SUARATO, Antonino, I-20158 Milan (IT)
(86) International application number: EP0006545
(87) International publication number: WO01005382

(56) References cited:
- WO-A-00/50032
- WO-A-00/50033
- WO-A-00/66093
- WO-A-99/20264
- WO-A-99/48503
- US-A- 4 853 221

## Description

The present invention relates in general to the field of cancer treatment and, more particularly, provides an antitumor composition comprising an alkylating anthracycline and an antimetabolite compound, having a synergistic or additive antineoplastic effect.

The present invention provides, in a first aspect, a pharmaceutical composition for use in antineoplastic therapy in mammals, including humans, comprising
- an alkylating anthracycline of formula Ia or Ib : 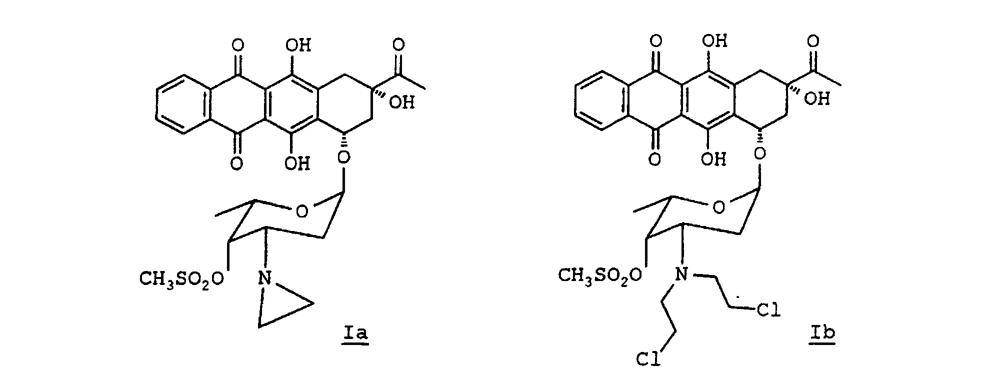
- an antimetabolite compound, and a pharmaceutically acceptable carrier or excipient.

The chemical names of the alkylating anthracyclines of formula Ia and Ib are 4-demethoxy-3'-deamino-3'-aziridinyl-4'-methansulfonyl daunorubicin (Ia) and 4-demethoxy-N,N-bis(2-chloroethyl)-4'-methansulfonyl daunorubicin (Ib). These alkylating anthracyclines were described in Anticancer Drug Design (1995), vol. 10, 641-653, and claimed respectively in US-A-5,532,218 and US-A-5,496,800. Both compounds intercalate into DNA via the chromophore and alkylate guanine at N⁷ position in DNA major groove via their reactive moiety on position 3' of the amino sugar. Compounds Ia and Ib are able to circumvent the resistance to all major classes of cytotoxics, indicating that the compounds represent a new class of cytotoxic antitumor drugs.

Antimetabolites are described in various scientific publications. The main representatives of this wide class of drugs are: the antifolates such as methotrexate, raltitrexed and trimetrexate; the 5-fluoropyrimidine compounds such as 5-fluorouracil, floxuridine and capecitabine; the cytidine analogs like cytarabine, azacitidine and gemcitabine. See for example the review: Cancer, Principles and Practice of Oncology, Lippincott-Raven Ed. (1997), 432-452 WO 99/20264 describes pharmaceutical formulations of antineoplastic drugs, such as alkylating agents, antimetabolites and anthracyclines, and a detoxylying agent. The 5-fluoropyrimidine compounds and the cytidine analogs are the preferred antimetabolite compounds to be used in the present invention, more preferably 5-fluorouracil or gemcitabine. The present invention also provides a product comprising an alkylating anthracycline of formula Ia or Ib as defined above and an antimetabolite compound, as combined preparation for simultaneous, separate or sequential use in antitumor therapy.

A further aspect of the present invention is to provide a medicament for treating a mammal including humans, suffering from a neoplastic disease state wherein an alkylating anthracycline of formula Ia or Ib as defined above and an antimetabolite compound, in amounts effective to produce a synergistic antineoplastic effect is to be administered to said mammal.

The present invention also provides a medicament for lowering the side effects caused by antineoplastic therapy with an antineoplastic agent in mammals, including humans, in need thereof, wherein a combination preparation comprising an antimetabolite compound as defined above and an alkylating anthracycline of formula Ia or Ib, as defined above, in amounts effective to produce a synergistic antineoplastic effect, is to be administered to said mammal. By the term "a synergistic antineoplastic effect" as used herein is meant the inhibition of the growth tumor, preferably the complete regression of the tumor, administering an effective amount of the combination of an alkylating anthracycline of formula Ia or Ib as defined above and a antimetabolite compound to mammals, including human.

By the term "administered " or "administering" as used herein is meant parenteral and /or oral administration. By "parenteral" is meant intravenous, subcutaneus and intramuscolar administration. In the invention, the alkylating anthracycline may be administered simultaneously with the compound with the antimetabolite compound activity, for example of the 5-fluoropyrimidine or cytidine class, or the compounds may be administered sequentially, in either order. It will be appreciated that the actual preferred method and order of administration will vary according to, inter alia, the particular formulation of the alkylating anthracycline of formula Ia or Ib being utilized, the particular formulation of the antimetabolite compound, such as one of the 5-fluoropyrimidine or cytidine class, being utilized, the particular tumor model being treated, and the particular host being treated.

In the invention, for the administration of the alkylating anthracycline of formula Ia or Ib, the course of therapy generally employed is from about 0.1 to about 200 mg/m² of body surface area. More preferably, the course therapy employed is from about 1 to about 50 mg/m-² of body surface area.

In the invention, for the administration of the antimetabolite compound the course of therapy generally employed is from about 0.1 to about 10 g/m² of body surface area. More preferably, the course therapy employed is from about 1 mg/m² to about 5 g/m² of body surface area. The antineoplastic therapy of the present invention is in particular suitable for treating breast, ovary lung, colon, kidney, stomach, pancreas, liver, melanoma, leukemia and brain tumors in mammals, including humans.

In a further aspect, the present invention is directed to the preparation of a pharmaceutical composition containing an effective amount of an alkylating anthracycline of formula Ia or Ib as defined above and an antimetabolite compound in the prevention or treatment of metastasis or for the treatment of tumors by angiogenesis inhibition, as well as to the use of an alkylating anthracycline of formula Ia or Ib as defined above and an antimetabolite compound for the treatment of tumors by angiogenesis inhibition or for the treatment or prevention of metastasis.

As stated above, the effect of an alkylating anthracycline of formula Ia or Ib and an antimetabolite compound, such as a 5-fluoropyrimidine or cytidine derivative, is significantly increased without a parallel increased toxicity. In other words, the combined therapy of the present invention enhances the antitumoral effects of the alkylating anthracycline and of the antimetabolites and thus yields the most effective and least toxic treatment for tumors.

The superadditive actions of the combination preparation of the present invention may be shown for instance by in vivo tests for the antileukemic activity on disseminated L1210 murine leukemia. The combination of Ia with gemcitabine (Table 1) or 5-Fluorouracil tested at the different doses and schedules, produces favorable ILS% values (Increase in life span: [(median survival time of treated mice/median survival time of controls)x 100]-100), indicating a synergistic effect.

Table 1 shows the antileukemic activity on disseminated L1210 murine leukemia obtained by combining the above PNU 159548 derivative with gemcitabine.

At the dose of 15 and 60 mg/kg of gemcitabine alone (ip day 1 after tumor injection) and at the dose of 1 and 1.5 mg/kg of PNU 159548 alone (iv day 1 after tumor injection, administered 2h after gemcitabine) were associated, without toxicity, with ILS% values of 50 and 83 and 33 and 67, respectively. By combining gemcitabine and PNU 159548 at the same doses and with the same schedule, an increase of activity with ILS% values of 117 and 204 were observed, indicating a synergistic effect as shown by the combination index (CI) of 1.4 and 1.3, respectively.

**Table 1:**

| | | | | | | |
|---|---|---|---|---|---|---|
| Antileukemic activity against disseminated L1210¹ murine leukemia of PNU-159548 (I) in combination with gemcitabine | | | | | | |

| Compound | Treatment schedule | Dose (mg/kg/day) | ILS%² | LTS³ | TOX⁴ | CI⁵ |
|---|---|---|---|---|---|---|
| PNU 159548 | iv +1(^{*}) | 1 | 33 | 0/10 | 0/10 | NA |
| | | 1.5 | 67 | 0/20 | 0/20 | NA |
| Gemcitabine | ip +1 | 15 | 50 | 0/10 | 0/10 | NA |
| | | 60 | 83 | 0/20 | 0/20 | NA |
| PNU 159548 + | iv +1(*) | 1 + 15 | 117 | 0/10 | 0/10 | 1.4 |
| gemcitabine | ip +1 | 1.5 + 60 | 204 | 4/20 | 2/20 | 1.3 |

| | | | | | | |
|---|---|---|---|---|---|---|
| 1. L1210 leukemia cells (10⁵/mouse CD2F1) are injected IV on Day 0. 2. Increase in life span: [(median survival time of treated mice/median survival time of controls) x 100] -100. | | | | | | |
| 3.LTS: long-term survivors (>60 days) at the end of the experiments | | | | | | |
| 4. Number of toxic deaths/number of mice. | | | | | | |
| 5. C.I. = combination Index : <1 antagonistic; 1 additive; >1 synergistic | | | | | | |
| (*)administered 2h after gemcitabine NA: not applicable | | | | | | |

For these experiments Ia was solubilized in [Cremophor® /EtOH = 6.5:3.5]/[normal saline]=20/80 v/v, while standard pharmaceutical preparation were used for the antimetabolite compounds.

## Claims

1. A product containing an alkylating anthracycline of formula Ia or Ib: 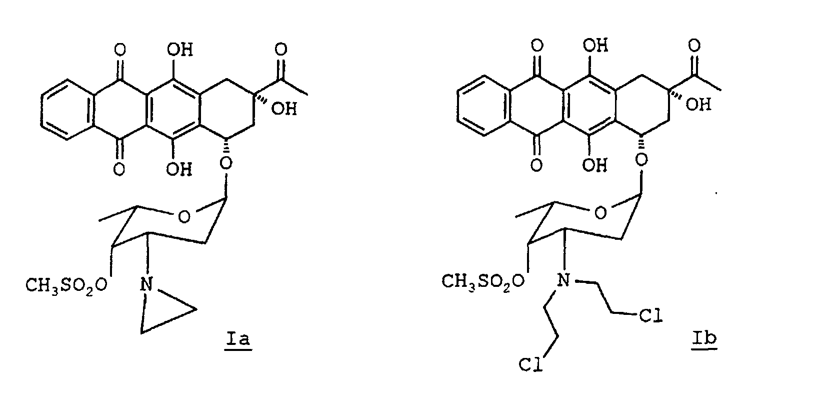 and an antimetabolite compound as a combined preparation for simultaneous, separate or sequential use in the treatment of tumors.

2. A product according to claim 1 wherein the alkylating anthracycline is 4-demethoxy-3'-deamino-3'-aziridinyl-4'-methanesulfonyl daunorubicin.

3. A product according to claim 1 or 2 wherein the antimetabolite compound is a cytidine analog.

4. A product according to claim 1 or 2 wherein the antimetabolite compound is a 5-fluoropyrimidine.

5. A product according to claim 3 wherein the cytidine analog is gemcitabine.

6. A product according to claim 4 wherein the 5-fluoropyrimidine is 5-fluorouracil.

7. A pharmaceutical composition comprising a pharmaceutically acceptable carrier or excipient and, as active ingredient, an alkylating anthracycline of formula Ia or Ib as defined in claim 1 and an antimetabolite compound.

8. A composition according to claim 7 wherein the antimetabolite compound is 5-fluorouracil or gemcitabine.

9. Use of an alkylating anthracycline of formula Ia or Ib as defined in claim 1 and an antimetabolite compound in the preparation of a medicament for use in the treatment of tumors.

10. Use according to claim 8 wherein the antimetabolite compound is 5-fluorouracil or gemcitabine.

11. Use of an alkylating anthracycline of formula Ia or Ib as defined in claim 1 and an antimetabolite compound in the preparation of a medicament for use in the prevention or treatment of metastasis or in the treatment of tumors by inhibition of angiogenesis.

## Patentansprüche

1. Produkt, enthaltend ein alkylierendes Anthracyclin der Formel (Ia) oder (Ib): 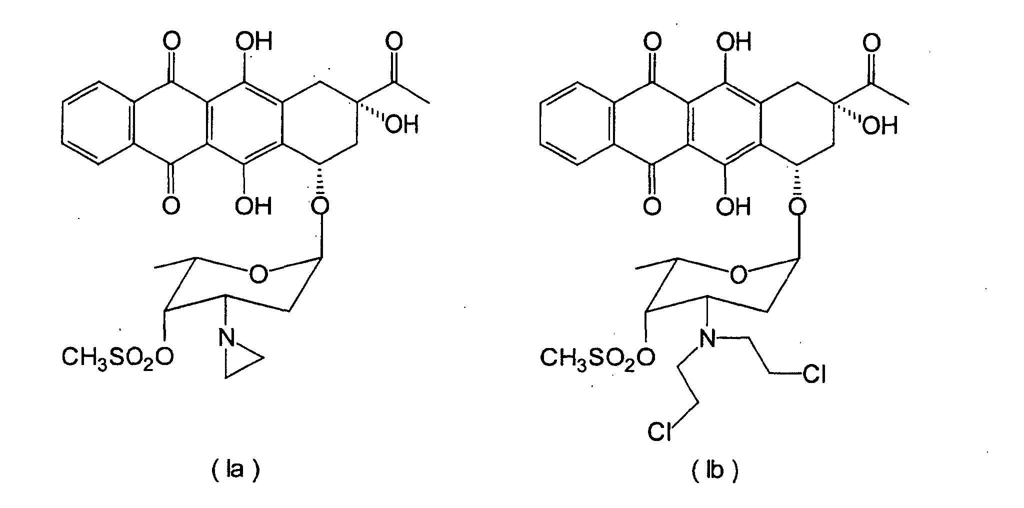 und eine Antimetabolitverbindung als Kombinationszubereitung zur gleichzeitigen, getrennten oder aufeinanderfolgenden Verwendung bei der Behandlung von Tumoren.

2. Produkt gemäss Anspruch 1, worin das alkylierende Anthracyclin 4-Desmethoxy-3'-desamino-3'-aziridinyl-4'methansulfonyldaunorubicin ist.

3. Produkt gemäss Anspruch 1 oder 2, worin die Antimetabolitverbindung ein Cytidinanalog ist.

4. Produkt gemäss Anspruch 1 oder 2, worin die Antimetabolitverbindung ein 5-Fluorpyrimidin ist.

5. Produkt gemäss Anspruch 3, worin das Cytidinanalog Gemcitabin ist.

6. Produkt gemäss Anspruch 4, worin das 5-Fluorpyrimidin 5-Fluoruracil ist.

7. Pharmazeutische Zusammensetzung, umfassend einen pharmazeutisch annehmbaren Träger oder Exzipienten und als Wirkstoff ein alkylierendes Anthracyclin der Formel (Ia) oder (Ib), wie in Anspruch 1 definiert, und eine Antimetabolitverbindung.

8. Zusammensetzung gemäss Anspruch 7, worin die Antimetabolitverbindung 5-Fluoruracil oder Gemcitabin ist.

9. Verwendung eines alkylierenden Anthracyclins der Formel (Ia) oder (Ib), wie in Anspruch 1 definiert, und einer Antimetabolitverbindung bei der Herstellung eines Arzneimittels zur Verwendung in der Behandlung von Tumoren.

10. Verwendung gemäss Anspruch 8, worin die Antimetabolitverbindung 5-Fluoruracil oder Gemcitabin ist.

11. Verwendung eines alkylierenden Anthracyclins der Formel (Ia) oder (Ib), wie in Anspruch 1 definiert, und einer Antimetabolitverbindung bei der Herstellung eines Arzneimittels zur Verwendung in der Prävention oder Behandlung von Metastasen oder in der Behandlung von Tumoren durch Inhibierung der Angiogenese.

## Revendications

1. Produit contenant une anthracycline alkylante de formule Ia ou Ib : 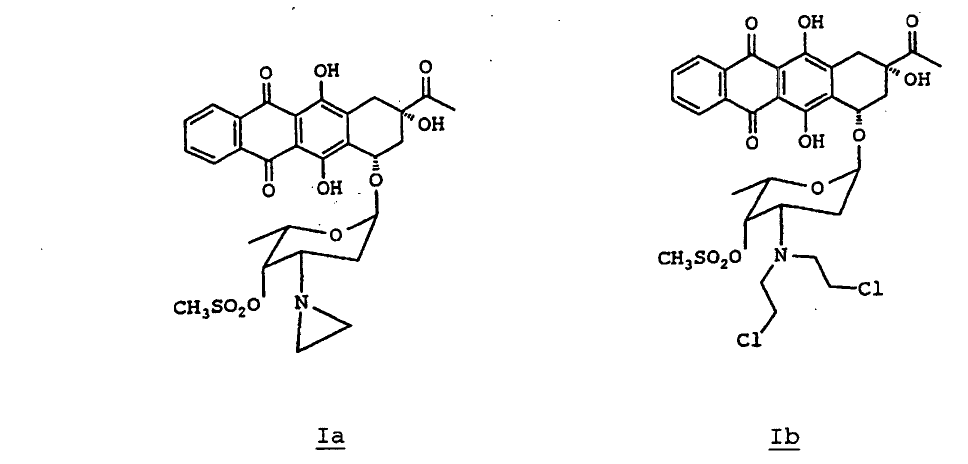 et un composé antimétabolite, en tant que préparation combinée pour une utilisation simultanée, séparée ou en séquence dans le traitement des tumeurs.

2. Produit selon la revendication 1, dans lequel l'anthracycline alkylante est la 4-déméthoxy-3'-désamino-3'-aziridinyl-4'-méthanesulfonyl daunorubicine.

3. Produit selon la revendication 1 ou 2, dans lequel le composé antimétabolite est un analogue de cytidine.

4. Produit selon la revendication 1 ou 2, dans lequel le composé antimétabolite est une 5-fluoropyrimidine.

5. Produit selon la revendication 3, dans lequel l'analogue de cytidine est la gemcitabine.

6. Produit selon la revendication 4, dans lequel la 5-fluoropyrimidine est le 5-fluorouracil.

7. Composition pharmaceutique comprenant un véhicule ou excipient pharmaceutiquement acceptable et, en tant qu'ingrédient actif, une anthracycline alkylante de formule Ia ou Ib telle que définie dans la revendication 1 et un composé antimétabolite.

8. Composition selon la revendication 7, dans laquelle le composé antimétabolite est le 5-fluorouracil ou la gemcitabine.

9. Utilisation d'une anthracycline alkylante de formule Ia ou Ib telle que définie dans la revendication 1 et d'un composé antimétabolite dans la préparation d'un médicament pour une utilisation dans le traitement des tumeurs.

10. Utilisation selon la revendication 8, dans laquelle le composé antimétabolite est le 5-fluorouracil ou la gemcitabine.

11. Utilisation d'une anthracycline alkylante de formule Ia ou Ib telle que définie dans la revendication 1 et d'un composé antimétabolite dans la préparation d'un médicament pour une utilisation dans la prévention ou le traitement de métastases ou dans le traitement de tumeurs par inhibition de l'angiogénèse.
